(19) Europäisches Patentamt
European Patent Office
Office européen des brevets

(11) Numéro de publication: 0 380 428 A1

(12) DEMANDE DE BREVET EUROPEEN

(21) Numéro de dépôt: 90420025.0

(22) Date de dépôt: 16.01.90

(51) Int. Cl.5: C08J 3/03, C08J 3/12, A61K 9/16, //C08L83:06

(30) Priorité: 19.01.89 FR 8900862

(43) Date de publication de la demande: 01.08.90 Bulletin 90/31

(84) Etats contractants désignés: AT BE CH DE DK ES FR GB GR IT LI LU NL SE

(71) Demandeur: RHONE-POULENC CHIMIE
25, quai Paul Doumer
F-92408 Courbevoie Cédex(FR)

(72) Inventeur: Demonceau, Daniel
80, Chemin de Chalamont
F-69140 Rillieux-La-Pape(FR)
Inventeur: Jorda, Rafael
30, rue Claude Jusseaud
F-69110 Sainte-Foy-Les-Lyon(FR)

(74) Mandataire: Seugnet, Jean Louis et al
RHONE-POULENC CHIMIE Service Brevets
Chimie Centre de Recherches des Carrières
B.P. 62
F-69192 Saint-Fons Cédex(FR)

# (54) Procédé de préparation de particules non collantes à base d'une composition organopolysiloxane réticulée par des réactions de polyaddition.

(57) La présente invention concerne un procédé de préparation de particules non collantes à base d'une composition organopolysiloxane réticulée par des réactions de polyaddition.

Selon ce procédé on met en émulsion les huiles silicones de départ en présence de poudre de silice et d'un catalyseur au platine, puis on casse l'émulsion et on réticule les particles par chauffage à une température de 40-100 °C. On obtient ainsi des particules non collantes, de granulométrie moyenne comprise entre 50 µm et 3 mm, revêtues de façon uniforme à leur surface de grains de silice.

Application du procédé de l'invention à l'encapsulation ou à la dispersion de principes actifs tels que médicaments ou produits phyto-sanitaires et à la préparation de charges souples pour matières plastiques synthétiques ou naturelles.

EP 0 380 428 A1

Xerox Copy Centre

# PROCEDE DE PREPARATION DE PARTICULES NON COLLANTES A BASE D'UNE COMPOSITION ORGANOPOLYSILOXANE RETICULEE PAR DES REACTIONS DE POLYADDITION

La présente invention concerne un procédé de préparation de particules non collantes à base d'une composition organopolysiloxane réticulée en un élastomère par des réactions de polyaddition, contenant éventuellement un principe actif encapsulé par l'élastomère et/ou dispersé au sein de l'élastomère.

Il existe une demande croissante de particules en élastomères silicones en vue de leur utilisation principalement dans deux types d'applications :

- l'incorporation de ces particules comme charge dans une matrice polymère en particulier dans une composition organopolysiloxane réticulable en un élastomère en vue notamment d'en augmenter la souplesse,
- le conditionnement (encapsulation ou dispersion) d'un principe actif par ces particules en vue de sa protection et éventuellement de sa libération contrôlée.

Parmi les nombreux documents brevets décrivant des procédés de préparation de particules ou de microparticles en élastomères silicones, réticulés par des réactions de polyaddition et/ou polycondensation, on peut citer plus particulièrement :

- EP-A-252 510 enseignant une procédé consistant à mélanger les divers constituants d'un élastomère silicone liquide, comportant éventuellement une charge siliceuse à une température comprise entre -60 °C et +5 °C et de les pulvériser ensuite dans un air chaud porté à une température comprise entre +80 °C et 200 °C, pour obtenir des particules de forme sensiblement sphérique, de granulométrie comprise entre quelques dizaines de millimicromètres à plusieurs centaines de micromètres.
- US-A-4 248 751 décrivant un procédé de préparation d'un latex silicone constitué de particules d'élastomère silicone polyaddition, dispersées dans une phase aqueuse qui consiste à polymériser en émulsion aqueuse un mélange d'huiles silicones vinylées, d'huiles silicones hydrogénées et d'un tensio-actif, puis à réticuler ces huiles par chauffage après addition d'un catalyseur au platine. Il est possible d'ajouter au latex une silice colloïdale afin d'améliorer les propriétés du revêtement élastomère filmogène obtenu après enduction du latex sur un support et évaporation de l'eau. Il n'est ni décrit, ni suggéré dans ce brevet l'obtention de particules d'élastomère silicone de granulométrie supérieure à 50 $\mu$m à partir de cette émulsion de latex.
- EP-A-217 257 décrivant un procédé de préparation de particules en silicone, de granulométrie comprise entre 0,01 et 10 mm qui consiste à polymériser en émulsion un diorganocyclopolysiloxane porteur de groupes vinyle et mercaptoalkyle, en présence d'une acide alkylarylsulfonique, à casser l'émulsion par addition de sulfate de magnésium et par chauffage, à introduire un monomère vinylique et à effectuer une polymérisation/greffage en présence d'initiateur radicalaire.
- US-A-4 594 134 enseignant l'obtention de microparticules ou particules sphériques, de granulométrie comprise entre 2 et 300 $\mu$m, en élastomère silicone polyaddition, obtenues par atomisation dans un séchoir vers 230 °C, d'une composition catalysée par du platine et renfermant un inhibiteur du platine. La réticulation peut être effectuée sans platine et sous U.V. mais avec un photosensibilisateur. Avant réticulation la composition peut être dispersée dans un milieu liquide tel qu'un solvant organique ou de l'eau.
- KOKAI 87/257 939 décrivant l'obtention de poudre de silicone par séchage et pulvérisation d'une émulsion aqueuse d'une composition silicone polyaddition.
- EP-A-267 003 visant la préparation de microsphères en élastomère silicone par dispersion dans l'eau en présence d'un tensio-actif, d'une composition organopolysiloxane réticulable par des réactions d'addition du type MICHAEL et réticulation de la composition.
- US-A-4 370 160 visant un procédé similaire à EP-A-267 003 sauf que la composition organopolysiloxane est réticulée sous un rayonnement U.V. en présence d'un photosensibilisateur.

Par rapport à l'état de la technique, la présente invention a principalement pour but de proposer un procédé de préparation de particules à base d'une composition organopolysiloxane réticulée en un élastomère par des réactions de polyaddition, qui permette d'aboutir directement à des particules qui soient stockables, facilement manipulables et donc qui ne s'agglomèrent pas les unes aux autres.

Un autre but de la présente invention est de proposer un procédé de préparation de particules, directement à partir de compositions organopolysiloxanes polyaddition disponibles dans le commerce.

Un autre but de la présente invention est de proposer un procédé de préparation de particules du type ci-dessus, ces particles pouvant être incorporées aisément et de façon homogène en tant que charge dans une matrice polymère synthétique ou naturelle, en particulier dans un élastomère silicone.

Un autre but de la présente invention est de proposer un procédé de préparation de particules en

élastomère silicone polyaddition qui permette directment soit d'encapsuler, soit de disperser de façon homogène au sein de l'élastomère, un principe actif quelconque en vue de sa protection et de son éventuelle libération contrôlée.

Ces buts et d'autres sont atteints par la présente invention qui concerne en effet un procédé de préparation de particules, de granulométrie moyenne comprise entre 50 µm et 3 mm, non collantes à base d'une composition organopolysiloxoane réticulée en un élastomère par des réactions de polyaddition, caractérisé en ce que :

a) - on met en émulsion :

(A) - 100 parties d'un polydiorganosiloxane portant par molécule au moins deux groupes Si-Vinyle, extrémité de sa chaîne par un motif vinyldiorganosiloxy,

(B) - un organohydrogénopolysiloxane présentant au moins trois groupes SiH par molécule en quantité telle que le rapport molaire SiH/SiVi soit compris entre 0,6 et 8, de préférence entre 0,8 et 4,

(C) - une quantité efficace d'un catalyseur au platine,

(D) - une quantité efficace d'un agent tensio-actif du type huile dans eau,

(E) - 3 à 100 parties, de préférence 5 à 30 parties d'une poudre de silice de combustion ou de précipitation,

(F) - de l'eau et, éventuellement

(G) - un principe actif.

b) - on forme les particules par chauffage à une température comprise entre 40 et 100 °C.

Selon une variante préférée, les organopolysiloxanes (A) et (B) sont introduits dans l'eau, sous la forme d'une solution dans un solvant organique volatil dont le point d'ébullition est inférieur à 100 °C, comme par exemple le chloroforme $CHCl_3$.

L'utilisation de solvant organique volatil procure plusieurs avantages :

- il permet de mettre facilement en émulsion aqueuse la composition organopolysiloxane même si cette dernière est une composition du commerce, se présentant en deux emballages avec ou sans charges déjà incorporées dans un des deux emballages,
- lors de l'étape b), le solvant organique s'évapore, l'émulsion aqueuse est alors cassée, ce qui facilite l'apparition des particules.

Le principe actif (G) est introduit dans l'émulsion de diverses manières :

- si le principe actif est soluble dans le solvant organique volatil, il peut être introduit en solution dans ce solvant,
- si le principe actif est soluble dans l'eau, il est souhaitable que l'eau de l'émulsion soit saturée en principe actif ; dans ce cas, le principe actif se trouve en majorité dispersé de façon homogène au sein des particules,
- si le principe actif (G) est insoluble dans l'eau et dans le solvant organique volatil, il est introduit sous forme de microparticules dispersées dans la composition organopolysiloxane, de préférence dans les huiles silicone (A) de départ, ces microparticules se trouvant encapsulées lors de l'étape b).

Après achèvement de l'étape b), les particules sont filtrées, éventuellement lavées par un alcool en particulier le méthanol, puis séchées pour éliminer l'eau résiduelle et achever, si nécessaire la réticulation de la composition organopolysiloxane.

Les particules sèches sont par la suite tamisées selon des zones de granulométrie prédéterminées pour éliminer l'excès éventuel de silice.

Le procédé de l'invention permet d'aboutir à des particules de granulométrie moyenne comprise entre 50 µm et 3 mm, plus spécifiquement entre 100 µm et 1 mm.

Le diorganopolysiloxane (A), de viscosité 100 à 300 000 mPa.s à 25 °C, de préférence 600 à 200 000 mPa.s à 25 °C, est un polymère linéaire, constitué d'une succession de motifs diorganosiloxy bloqués en bout de chaîne par un motif vinyldiorganosiloxy. Les radicaux organiques liés aux atomes de silicium du polymère sont choisis de préférence parmi les radicaux méthyle, éthyle, n-propyle, phényle, trifluoro-3,3,3 propyle. De préférence, au moins 90 % molaire de ces radicaux sont des radicaux méthyle, au plus 10 % molaire sont des radicaux phényle.

A titre d'exemples concrets de motifs diorganosiloxy, peuvent être cités ceux de formules : $(CH_3)_2SiO$, $CH_3(C_6H_5)SiO$, $(C_6H_5)_2SiO$, $CH_3(n.C_3H_7)SiO$, $CH_3(C_6H_5)SiO$.

De préférence on utilise une huile diméthylpolysiloxane bloquée à chaque extrémité de sa chaîne par un motif diméthylvinylsiloxy ou un motif méthylphénylvinylsiloxy, de viscosité 300 à 150 000 mPa.s à 25 °C.

L'huile vinylée (A) définie ci-dessus est commercialisée par les fabricants de silicones.

De façon à améliorer les propriétés mécaniques de l'élastomère il est recommandé d'incorporer en outre comme organopolysiloxane (A) une résine organopolysiloxane solide à température ambiante,

comportant des motifs M et Q respectivement de formules $R_3SiO_{0,5}$ et $SiO_2$, dans lesquelles R est choisi parmi les radicaux méthyle, éthyle, n-propyle, phényle et vinyle, résine dans laquelle d'une part le rapport molaire M/Q est compris entre 0,5 et 1, et d'autre part, 1,5 à 10 moles d'atomes de silicium comportent un radical vinyle directement lié à l'atome de silicium.

Cette résine peut comporter en outre un motif D de formule $R_2SiO$, R ayant la signification ci-dessus, de telle sorte qu'il reste de 1 à 10 moles de motif D par rapport au nombre total de motifs MDQ présents dans la résine.

L'organohydrogénosiloxane (B) peut être un polymère linéaire, cyclique ou ramifié ou ses mélanges contenant des motifs siloxane de formules : $R'SiO_{1,5}$, $R'_2SiO$, $R'_3SiO_{0,5}$, $R'_2HSiO_{0,5}$, $SiO_2$, $R'HSiO$ et $HSiO_{1,5}$, dans lesquelles le radical R' a la même signification que celle donnée ci-dessus pour les radicaux organiques du diorganopolysiloxane (A).

Ils sont par exemple décrits dans les brevets US-A-2 486 162, US-A-3 284 406 et US-A-3 436 366.

Selon un mode particulier de réalisation de l'invention et dans le but d'obtenir des particules d'élastomère silicone présentant une hydrophilie et/ou une organophilie améliorée, on remplace tout ou partie du diorganopolysiloxaneo (A) par un diorganopolysiloxane ($A_1$) présentant en outre, par rapport au diorganopolysiloxane (A), au moins un radical organique époxy fonctionnel ayant de 4 à 20 atomes de carbone.

On peut également remplacer tout ou partie de l'organohydrogénopolysiloxane (B) par un organohydrogénopolysiloxane ($B_1$) présentant en outre, par rapport au diorganopolysiloxane (B) au moins un radical organique époxy fonctionnel ayant de 4 à 20 atomes de carbone.

On peut également remplacer tout ou partie des polymères (A) et (B) par des polymères ($A_1$) et ($B_1$).

Les diorganopolysiloxanes ($A_1$) sont plus particulièrement choisis parmi ceux de formule :

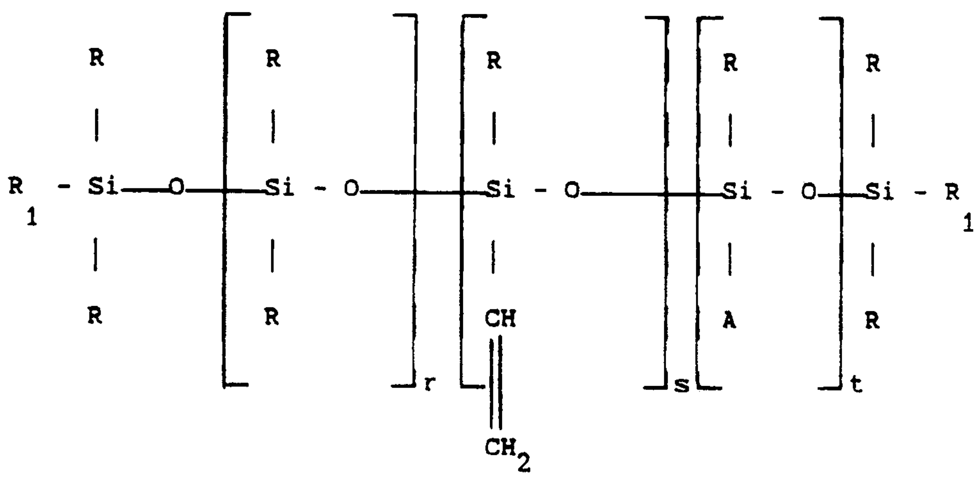

(1)

dans laquelle :

- les radicaux R sont des radicaux hydrocarbonés monovalents choisis parmi les radicaux alkyle en $C_1$-$C_{20}$, phényle, méthyle et trifluoro-3,3,3 propyle,
- les radicaux $R_1$, identiques ou différents sont choisis parmi un radical R, un radical A, un radical vinyle et un radical hydroxyle,
- le radical A est un radical organique époxy-fonctionnel ayant de 4 à 20 atomes de carbone,
- r est un nombre entier compris entre 1 et 500,
- s est un nombre entier compris entre 0 et 200,
- t est un nombre entier compris entre 0 to 100,

sous réserve que si S = 0, les deux radicaux $R_1$ sont un radical vinyle, et t est au moins égal à 1, si t = 0, au moins un des radicaux $R_1$ est un radical A et s est au moins égal à 2.

Dans la formule (1) ci-dessus, les produits préférés de formules (1) et (2) sont ceux pour lesquels au moins 80 % et jusqu'à 100 % en nombre des radicaux R sont des radicaux méthyle.

Le radical organique époxy-fonctionnel présente de 4 à 20 atomes de carbone, de préférence de 5 à 12.

Des exemples de radicaux A sont les suivants :

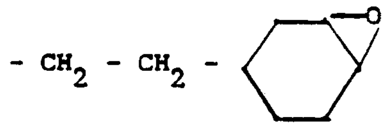

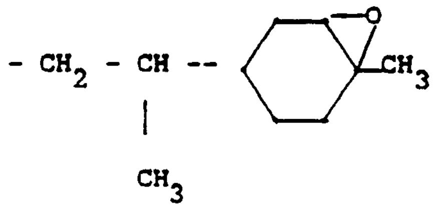

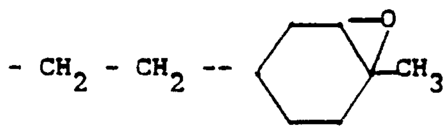

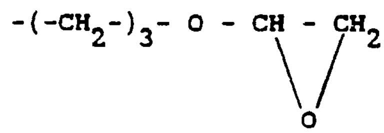

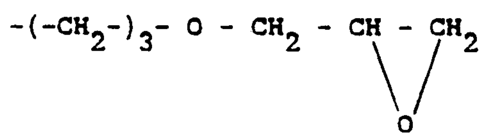

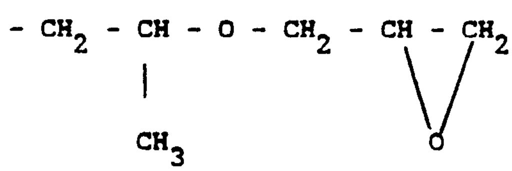

De préférence dans la formule (1) s et t sont au moins égaux à 1, r est compris entre 5 et 300 inclus.

De façon plus préférée s est compris entre 2 et 20 inclus, t est compris entre 1 et 30 inclus et r est compris entre 5 et 100 inclus.

Les polydiorganosiloxanes ($A_1$) peuvent être préparés de la façon suivante :

- on amorce, dans une première étape, la polymérisation d'au moins un oligomère polydiorganosiloxane cyclique ou linéaire portant par molécule au moins un radical vinyle lié directement à l'atome de silicium en présence d'une quantité catalytiquement efficace d'un catalyseur basique, et
- on ajoute, dans une deuxième étape, dans l'intervalle de temps situé entre le début et la fin de la polymérisation de la première étape, au moins un oligomère polydiorganosiloxane cyclique ou linéaire portant par molécule au moins un radical organique époxy-fonctionnel ayant de 4 à 20 atomes de carbone.

Ce procédé de préparation est décrit en détail dans la demande de brevet français 88/04 138 déposée le 24 mars 1988 au nom de la Demanderesse et citée comme référence.

Les oligomères linéaires ou cycliques porteurs d'un groupe vinyle sont connus. Ils sont en particulier décrits dans l'ouvrage de NOLL, Chemistry and Technology of Silicones, Academic Press édition de 1968 en langue anglaise, ainsi que dans de nombreux brevets (US-A-3 220 972, US-A-3 284 406 et US-A-3 436 366.

On peut utiliser dans le procédé de préparation des polymères ($A_1$) les différents catalyseurs alcalins dont l'emploi est préconisé pour la polymérisation d'oligomères diorganopolysiloxanes (c'est-à-dire les diorganopolysiloxanes de faible masse moléculaire et de faible degré de polymérisation).

Ces catalyseurs alcalins sont par exemple décrits à la page 227 de l'ouvrage de NOLL "Chemistry and Technology of Silicones" Academic Press, édition 1968 en langue anglaise. Ces catalyseurs alcalins englobent les hydroxydes de métaux alcalins, en particulier l'hydroxyde de lithium, l'hydroxyde de potassium, l'hydroxyde de sodium, l'hydroxyde de cesium. Ils englobent également les silanolates de métaux alcalins, en particulier de potassium ou de sodium, les alcoxydes et les siliconates de métaux alcalins.

De façon à accélérer la réaction, il est avantageux d'utiliser en combinaison avec le catalyseur alcalin un agent séquestrant le cation du catalyseur, à la dose de 0,005 à 2 moles d'agent séquestrant par équivalent hydroxyde de métal alcalin ou alcalino-terreux.

Comme agents séquestrants utilisables dans l'application on peut notamment utiliser les éther-couronnes ("crown-ethers") selon l'enseignement des brevets US-A-4 157 337 et US-A-4 138 543, cités comme référence et les tris(oxoalkyl) amine selon l'enseignement des brevets US-A-4 362 855 et EP-A-180 527.

Parmi ces agents séquestrants on préfère tout particulièrement utiliser un composé polyhétéromacropolycyclique cryptand renfermant 2 atomes d'azote tertiaire reliés entre eux par 3 radicaux organiques hydrocarbonés divalents renfermant des hétéroatomes choisis parmi l'oxygène et le soufre. Ces cryptands sont décrits en détail dans les brevets US-A-4 138 543 et US-A-4 362 855 précités.

Les cryptands préférés répondent à la formule :

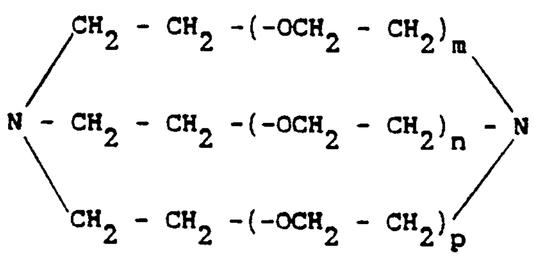

(2)

dans laquelle m est égal à 2 ou 3 et n et p sont choisis parmi 1, 2 et 3.

Le produit de formule (2) pour lequel m = n = p = 2 est nommé cryptand (2,2,2).

Le système destiné à catalyser la réaction de polymérisation selon l'invention, peut être utilisé en proportions très variées. Il se révèle particulièrement remarquable du fait qu'il peut être mis en oeuvre en très faible quantité. Habituellement, on utilise de 0,1 à 1 000 mg et préférentiellement de 1 à 100 mg d'équivalent d'hydroxyde de métal alcalin ou alcalino-terreux par kg de diorganopolysiloxane chargé. L'équivalent d'hydroxyde pour un dérivé alcalin ou alcalino-terreux quelconque est exprimé par le poids de la quantité d'hydroxyde du métal que l'on devrait ajouter et qui correspondrait au même nombre d'atomes grammes de métal alcalin ou alcalino-terreux que celui du dérivé alcalin ou alcalino-terreux utilisé. Par ailleurs, le rapport molaire agent séquestrant sur équivalent hydroxyde de métal alcalin ou alcalino-terreux est habituellement compris entre 0,005 et 2 et préférentiellement entre 0,01 et 1.

La température de la polymérisation peut se situer dans la gamme usuelle de température comprise entre 25 °C et 200 °C et de préférence entre 80 °C et 180 °C. Durant la polymérisation, l'eau est éventuellement éliminée du mileu réactionnel (dans le cas où la charge de départ est formée d'oligomère de formule (3) avec $R_2$ = hydroxyle), au fur et à mesure de sa formation par tout moyen approprié (distillation azéotropique ; élimination de l'eau sous pression réduite et sous pression de gaz inerte en particulier l'azote ...). Dans ces conditions, le système catalytique selon l'invention permet de réduire dans une proportion très importante la durée de la réaction de polymérisation. Par rapport au catalyseur alcalin utilisé seul, le gain peut atteindre et dépasser 75 %. Lorsque la viscosité souhaitée est atteinte, il est recommandé de désactiver le système catalytique par addition d'une très faible quantité d'un composé acide, de préférence du $CO_2$.

Dès l'instant où la polymérisation est amorcée (temps $t_1$) et avant de stopper cette polymérisation au degré voulu (temps $t_2$), on ajoute entre $t_1$ et $t_2$ l'oligomère polydiorganosiloxane cyclique ou linéaire portant par molécule au moins un radical époxy-fonctionnel.

Les temps $t_1$ et $t_2$ sont appréciés classiquement par mesure de la viscosité du mélange réactionnel.

De façon surprenante et inattendue, les fonctions époxy ne sont pas affectées par les catalyseurs basiques dans la mesure où les oligomères sont ajoutés entre $t_1$ et $t_2$.

Ces fonctions époxy ne sont pas dégradées malgré l'alcalinité du mélange réactionnel.

De plus, les fonctions époxy ne sont pas dégradées même si le milieu réactionnel est porté jusqu'à 200 °C. Il est toutefois souhaitable d'opérer à une température inférieure à 180 °C et supérieure à 80 °C.

Un tel résultat peut être aussi atteint si on ajoute les oligomères, porteurs d'au moins un radical époxy-fonctionnel aux oligomères porteurs d'au moins un groupe vinyle avant de commencer (amorcer) la polymérisation. Par contre on constate une dégradation des fonctions époxy. Il en est de même si on utilise un catalyseur acide pour faire la polymérisation. Les oligomères porteurs d'au moins un radical organique époxy-fonctionnel peuvent être en particulier choisis parmi ceux de formule :

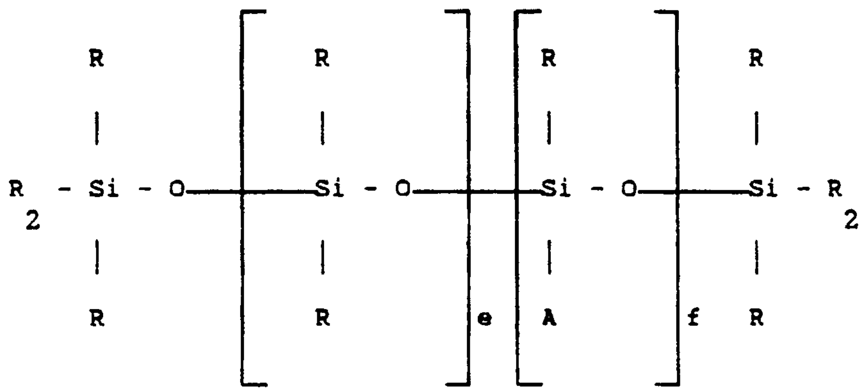

(3)

dans laquelle les radicaux R et A ont la même signification qu'à la formule (1) ci-dessus et $R_2$ est choisi parmi un radical R, un groupe hydroxyle et un groupe A,
- e est un nombre entier choisi entre 0 et 50,
- f est un nombre entier choisi entre 0 et 20,

sous réserve que si f = 0, au moins un des radicaux $R_2$ est A.

Et ceux de formule :

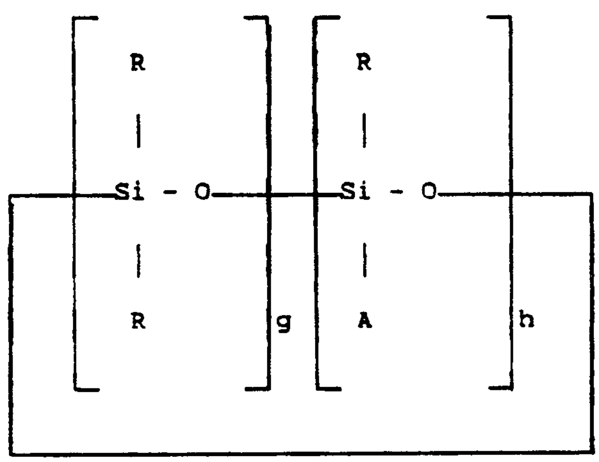

(4)

dans laquelle les radicaux R et A ont la signification donnée à la formule (1) ci-dessus,
- g est un nombre entier compris entre 0 et 10 inclus, et
- h est un nombre entier compris entre 1 et 10 inclus sous réserve que la somme g + h soit au moins égale à 3.

Les oligomères à fonction époxy sont des produits connus et décrits dans la littérature ; ils sont en particulier décrits dans les brevets US-A-3 455 877, US-A-4 252 933 et FR-A-2 012 012, cités comme référence.

Ils peuvent être préparés sans difficulté par réaction d'hydrosilylation des polyhydrogénoorganosiloxanes correspondants aux formules (3) et (4) dans lesquelles le radical A est remplacé par un atome d'hydrogène, sur le composé organique époxy-fonctionnel à insaturation alcénique correspondant au radical A.

L'homme de l'art sait choisir les oligomères de départ à fonction vinyle ou époxy en vue d'obtenir les polymères désirés de formule (1).

Dans le cas où l'on veut obtenir des produits de formule (1) dans laquelle $R_1$ est un radical R, il suffit pour cela, après la polymérisation, d'ajouter par exemple un composé organosilicié susceptible de réagir

avec les hydroxyle terminaux par exemple un silane ou un silazane de formule :
$(R)_3SiCl$ , $(R)_3SiNHSi(R)_3$ ,
ou d'ajouter en fin de polymérisation un bloqueur de formule :
$(R)_3SiOSi(R)_3$

De la même façon si l'on veut obtenir un polymère de formule (1) dans laquelle $R_1$ est un radical vinyle ou un radical A il suffit d'effectuer les mêmes opérations que ci-dessus avec un silane ou un silazane de formule :
$R_1(R)_2SiCl$ , $R_1(R)_2SiNHSi(R)_2R_1$
ou un disiloxane de formule :
$R_1(R)_2$ Si - O - Si $(R)_2$ $R_1$
$R_1$ désignant respectivement un radical vinyle ou le radical A.

La réaction de polymérisation est de préférence mise en oeuvre en masse, toutefois il est tout à fait possible d'utiliser un solvant tel que les hydrocarbures aliphatiques ou aromatiques.

L'organohydrogénopolysiloxane ($B_1$) utilisable est bien connu et est décrit en particulier dans les brevets précités US-A-2 915 497, US-A-3 284 406, US-A-3 436 366 et US-A-3 699 073.

L'organohydrogénosiloxane ($B_1$) peut être un polymère linéaire, cyclique ou ramifié ou ses mélanges contenant des motifs siloxanes de formules : $RSiO_{1,5}$, $R_2SiO$, $R_3SiO_{0,5}$, $R_2HSiO_{0,5}$, $SiO_2$, RHSiO et $HSiO_{1,5}$.

Le catalyseur (C) de la réaction de polyaddition est un métal ou un composé (sel ou complexe) à base de platine.

Les catalyseurs au platine sont amplement décrits dans la littérature, on peut en particulier citer les complexes du platine et d'un produit organique décrit dans les brevets américains US-A-3 159 601, US-A-3 159 602, US-A-3 220 972 et les brevets européens EP-A-57 459, EP-A-188 978 et EP-A-190 530 et les complexes du platine et d'organopolysiloxane vinylé décrits dans les brevets américains US-A-3 419 593, US-A-3 715 334, US-A-3 377 432 et US-A-3 814 730.

Le catalyseur au platine (C) est généralement introduit de manière à fournir de 1 à 500 ppm (partie par million), de préférence de 5 à 80 ppm de platine, exprimé en métal par rapport au poids total des organopolysiloxanes (A).

Les émulsions selon l'invention sont du type huile dans l'eau, plus particulièrement silicone dans l'eau (émulsion silicone/eau). Les émulsions contiennent une quantité efficace d'au moins un agent tensio-actif (D) choisi parmi les agents tensio-actifs anioniques, cationiques, amphotères et non-ioniques. De tels agents tensio-actifs sont par exemple décrits dans les brevets US-A-2 891 920, US-A-3 294 725, US-A-3 360 491, US-A-3 983 148 et FR-A-2 605 634 cités comme références.

Les agents tensio-actifs anioniques préférés sont choisis parmi les alkylbenzène sulfonates de métaux alcalins, les sulfates d'alkyle (ou d'alkylaryle éthoxylés) et de métaux alcalins et les dioctylsulfosuccinates de métaux alcalins.

Les agents tensio-actifs cationiques préférés sont choisis parmi les chlorures d'ammonium quaternaire et les sels d'ammonium quaternaire polyéthoxylés.

Les agents tensio-actifs non-ioniques préférés sont choisis parmi les acides gras polyéthoxylés, les esters de sorbitan, les esters de sorbitan polyéthoxylés, les alkylphénols polyéthoxylés et les alcools polyvinyliques.

On utilise généralement de 1 à 30 parties, de préférence de 2 à 10 parties, de tensio-actif (D) pour 100 parties de la totalité des polymères (A) et (B) organopolysiloxane.

Un tensio-actif convenant plus particulièrement est l'alcool polyvinylique. C'est un produit solide granuleux qui peut encore présenter des groupes acétate. Généralement son pourcentage d'hydrolyse est élevé, dépassant 85 %. On peut utiliser par exemple le RHODOVIOL ® 25/140 de RHONE-POULENC, ou l'ELVANOL ® 50-42 de DUPONT. On utilise généralement de 1 à 20 parties, de préférence de 2 à 10 parties d'alcool polyvinylique pour 100 parties de la totalité des polymères organopolysiloxanes (A) et (B).

Les silices (E) sont choisies parmi les silices de combustion et les silices de précipitation.

Elles ont une surface spécifique, mesurée selon les méthodes BET, d'au moins 50 $m^2/g$, de préférence supérieur à 70 $m^2/g$, une dimension moyenne des particules primaires inférieure à 80 nanomètre et une densité apparente inférieure à 200 g/litre.

Ces silices peuvent être incorporées telles quelles ou après avoir été traitées par des composés organosiliciques habituellement utilisés pour cet usage. Parmi ces composés, figurent les méthylpolysiloxanes tels que l'hexaméthyldisiloxane, l'octaméthylcyclotétrasiloxane, des méthylpolysilazanes tels que l'hexaméthyldisilazane, l'hexaméthylcyclotrisilazane, des chlorosilanes tels que le diméthyldichlorosilane, le triméthylchlorosilane, le méthylvinyldichlorosilane, le diméthylvinylchlorosilane, des alcoxysilanes tels que le diméthyldiméthoxysilane, le diméthylvinyléthoxysilane, le triméthylméthoxysilane. Lors de ce traitement, les silices peuvent accroître leur poids de départ jusqu'à un taux de 20 %, de préférence 18 % environ.

Les particules préparées par le procédé de l'invention sont utilisables pour enrober, encapsuler ou disperser dans une matrice d'élastomère, un principe actif quelconque tel que, notamment, des adhésifs, des catalyseurs, des colorants, des durcisseurs, des détergents, des produits pharmaceutiques, des enzymes, des parfums, des aliments, des combustibles, des encres, des insecticides, des métaux, des médicaments, des monomères, des agents odorants, des huiles, des phéromones, des plastifiants, des propulseurs, des solvants, des substrats solides contenant un constituant actif absorbé et des vitamines.

Les émulsions selon l'invention peuvent être préparées selon les méthodes classiques de mise en émulsion de polymères organopolysiloxanes.

On peut par exemple mélanger l'agent tensio-actif (D) et la poudre de silice (E) à de l'eau à une température comprise entre la température ambiante et 80 °C jusqu'à l'obtention d'un mélange homogène. On ajoute alors les organopolysiloxanes (A) et (B) au mélange tout en agitant énergiquement toujours à une température comprise entre 25 et 80 °C, pour former une émulsion. Les organopolysiloxanes (A) et (B) peuvent être mis au préalable en solution dans un solvant organique.

Les particules obtenues par le procédé de l'invention contenant ou non un principe actif (G) sont plus particulièrement utilisables comme charge, dans les matrices polymères naturels ou synthétiques, en particulier dans les caoutchoucs naturels ou synthétiques et les élastomères silicones.

On ajoute alors le catalyseur au platine (C) et le principe actif (G).

Si le principe actif (G) est soluble dans l'eau et insoluble dans le solvant organique volatil, on peut l'introduire dans l'eau de départ de l'émulsion, de préférence à saturation.

Si le principe actif (G) est soluble dans le solvant organique volatil et insoluble sand l'eau, on peut l'introduire dans la solution du solvant organique volatil des polyorganosiloxanes (A) et (B).

Si le principe actif (G) est insoluble dans l'eau et dans le solvant organique volatil, on peut l'introduire sous forme de microparticules et le disperser dans la composition organopolysiloxane avant sa mise en émulsion, de préférence seulement dans l'huile (A) de départ. Cer dernier mode d'introduction de (G) est bien entendu utilisable quelle que soit la solubilité de (G) dans l'eau et dans le solvant organique volatil.

Puis on porte l'émulsion à une température (inférieure à 100 °C) et pendant une durée adaptée à la formation des particules solides.

On refroidit ensuite la solution à température ambiante, on isole les particules par centrifugation ou par filtration sur toile polyester et on lave plusieurs fois à l'eau pour éliminer le tensio-actif et la silice.

Après un lavage éventuel par un alcool, de préference le méthanol, il peut être avantageux de mélanger les particules obtenues par de la silice en poudre pour parfaire leur caractère non collant, puis de tamiser les particules pour éliminer la poudre de silice en excès.

De façon tout à fait surprenante et inattendue, la présente invention a permis de mettre en évidence que les grains de silice se trouvent concentrés à la surface des particules ce qui leur confère leur caractère non collant. Les particules se présentent sous la forme d'une poudre dont les différents grains ne s'agglomèrent pas entre eux.

Les exemples suivants illustrent l'invention sans en limiter la portée.

Dans ce qui suit ou ce qui précède, sauf indication contraire, les parties et pourcentages sont en poids.

- EXEMPLE 1:

On prépare une solution aqueuse contenant :

- 1 200 g d'eau distillée,
- 5 g de tensio-actif RHODOVIOL ® 25/140, commercialisé par la Société RHONE-POULENC,
- 11,2 g de silice de précipitation de surface spécifique BET 160 $m^2/g$.

On coule dans cette solution aqueuse, sous forte agitation pour former une émulsion, 100 g d'une composition d'organopolysiloxane provenant du mélange de :

- 694 parties d'une huile vinylée polydiméthylsiloxane bloquée à chacune de ses extrémités par un groupement vinyldiméthylsiloxy de viscosité 3 500 mPa.s à 25 °C,
- 261 parties d'excipients secs, d'une résine vinylée présentant 53,5 % molaire de motifs $SiO_2$, 6 % molaire de motifs $(CH_3)(CH_2=CH)SiO$ et 40 % molaire de motifs $(CH_3)_3SiO_{0,5}$,
- 45 parties d'une résine silicone liquide hydrogénée préparée par hydrolyse de silicate technique $Si(OC_2H_5)_4$ et de $(CH_3)_2HSiCl$ en des quantités correspondant à 1 mole de $SiO_2$ pour 2 moles de $(CH_3)_2HSiCl$ en solutions dans le toluène. Cette résine présente donc un rapport molaire théorique de motifs $(CH_3)HSiO_{0,5}/SiO_2 = 2$ et un rapport molaire réel de 2,23,
- 0,02 partie (calculée en platine métal) d'une solution catalytique préparée par mélange à température ambiante de 0,6 partie d'acide chloroplatinique, 10 parties d'isopropanol, 55 parties de xylène et 6 parties

de tétraméthyl-1,1,3,3 divinyl-1,3 disiloxane.

On porte la température de l'eau à 50 °C ; on l'y maintient 15 minutes puis on la monte progressivement jusqu'à 80 °C et on laisse sous agitation pendant 1 heure 30 minutes. Ensuite, on refroidit les solutions à 25 °C, on filtre les particules sur toile polyester et on lave plusieurs fois à l'eau pour éliminer le tensio-actif et la silice.

Ces particules sont récupérées et mélangées à 4 parties de la même silice de précipitation utilisée au départ, puis placées sous vide à 125 °C pendant 3 heures. Elles sont ensuite récupérées, tamisées pour éliminer l'excès de silice et fractionnées par taille de particules.

Le rendement massique obtenu est de 90 %.

Par passage au tamis, on détermine la répartition suivante de diamètre particulaire $\phi$ des particules :
200 $\mu$m < $\phi$ < 500 $\mu$m : 40 % en poids
500 $\mu$m < $\phi$ < 1 000 $\mu$m : 60 % en poids.

## - EXEMPLE 2 :

On prépare une solution aqueuse contenant :
- 600 g d'eau distillée,
- 3 g de tensio-actif MONTANOX ® 20 (monolaurate sorbitan polyéthoxyl, commercialisé par la Société SEPPIC),
- 4,7 g de silice de précipitation de surface spécifique BET 160 $m^2/g$,
- 343 g de KCl.

On coule dans cette solution aqueuse à 40 °C, sous forte agitation pour former une émulsion, 42 parties de la composition organopolysiloxane définie à l'exemple 1, 18g de KCl et 15 g de $CHCl_3$.

Après 1 heure 30 minutes d'agitation, on monte la température à 60 °C, on la maintient pendant 30 minutes à cette température avant de l'amener à 80 °C pour y rester pendant 2 heures.

On refroidit ensuite la solution à 25 °C et on filtre pour récupérer les particules et la silice. L'ensemble est placé sous vide à 125 °C pendant 3 heures. On récupère le produit et on tamise pour éliminer la silice en excès.

Les particules obtenues ont un taux d'extractibles de 4,5 % par rapport au poids de composition organopolysiloxane, une taille moyenne de 700 $\mu$m et un taux de charge en KCl de 15 % en poids.

## - EXEMPLE 3 :

On prépare une solution aqueuse contenant :
- 600 parties d'eau distillée,
- 2,5 parties de RHODOVIOL ʳ 25/140,
- 45,2 parties de $KIO_3$,
- 4,7 parties de silice de précipitation de surface spécifique BET 160 $m^2/g$.

On coule dans cette solution aqueuse maintenue à 25 °C sous forte agitation pour former une émulsion, 42 parties de la composition organopolysiloxane définie à l'exemple 1, 18 g de KCl et 15 g de $CHCl_3$.

On monte progressivement la température à 50 °C et on laisse sous agitation durant 15 minutes, ensuite on monte progressivement la température à 80 °C et on laisse sous agitation pendant 1 heure 30 minutes.

On refroidit la solution à 25 °C et on filtre pour récupérer les particules et la silice. L'ensemble est placé sous vide à 60 °C sous vide pendant 12 heures. On récupère le produit et on tamise pour éliminer la silice en excès.

Les particules obtenues ont un taux d'extractibles de 5,5 % par rapport au poids de composition organopolysiloxane.

La répartition de taille des particules obtenues est la suivante :
200 $\mu$m < $\phi$ < 500 $\mu$m : 40 % en poids
500 $\mu$m < $\phi$ < 1 000 $\mu$m : 60 % en poids.

La teneur en KCl dispersé dans les particules est de 22 % en poids.

## - EXEMPLE 4 :

On prépare une solution aqueuse contenant :

- 600 ml d'eau distillée,
- 2,5 g de tensio-actif RHODOVIOL ® 25/140 (alcool polyvinylique, commercialisé par la Société RHONE-POULENC,
- 5,6 g de silice de précipitation TIXOSIL ® 375, commercialisé par la Société RHONE-POULENC.

On ajoute à ce mélange, séparément et dans l'ordre :

- 50 g de la composition organopolysiloxane définie à l'exemple 1. Cette composition étant ajoutée à la solution aqueuse sous forte agitation pour former une émulsion,
- 21,4 g d'un mélange de carboxyméthylcellulose et de VANTOCIL ® 1B ayant la composition suivante :
- carboxyméthylcellulose : 12,4 g
- VANTOCIL 1B : 9,0 g

Ce mélange se présente sous la forme de particules solides, de granulométrie moyenne inférieure à 10 µm,

- 17,3 g de $CHCl_3$

On porte le mélange obtenu à une température de 50 °C pendant 15 minutes. Cette température est montée progressivement à 80 °C et est maintenue pendant 1 heure 30 minutes, le mélange étant agité. On refroidit le mélange à 25 °C.

Les billes formées sont filtrées sur une toile en polyester puis lavées plusieurs fois à l'eau pour éliminer le tensio-actif et la silice.

Ces billes sont récupérées et mélangées à 2 g de silice TIXOSIL T375 puis placées sous vide à 125 °C durant 3 heures. Elles sont ensuite récupérées, tamisées pour éliminer l'excès de silice et les fractionner par taille.

Le rendement massique obtenu est de 93 % et la granulométrie des billes est la suivante :

$\phi$ > 1 mm : 25,1 %
500 µm < $\phi$ < 1 mm : 29,3 %
200 µm < $\phi$ < 500 µm : 26,8 %
100 µm < $\phi$ < 200 µm : 14,1 %
$\phi$ < 100 µm : 25,1 %

Les microbilles obtenues ont un taux d'extractibles de 5 % par rapport à la masse de la composition organopolysiloxane introduite.

## - EXEMPLE 5 :

On procède exactement comme à l'exemple 4 sauf que l'on ajoute à la solution aqueuse 2,5 g d'un tensio-actif supplémentaire, type anionique, à savoir du dodécyl sulfate de sodium (lauryl sulfate de sodium).

Le rendement massique est de 70 % environ, on obtient la répartition suivante de granulométrie des billes de silicone obtenues :

$\phi$ > 1 mm : 0 %
500 µm < $\phi$ < 1 mm : 7,6 %
200 µm < $\phi$ < 500 µm : 53,3 %
100 µm < $\phi$ < 200 µm : 28,2 %
$\phi$ < 100 µm : 10,9 %

Ainsi, l'ajout d'un tensio-actif anionique lauryl sulfate de sodium, permet d'obtenir des microbilles présentant une meilleure monodispersité suivant une granulométrie comprise, essentiellement, entre 100 et 500 µm.

Les microbilles obtenues ont un taux d'extractibles de 4,3 % par rapport à la masse de la composition organopolysiloxane introduite.

## - EXEMPLE 6 :

On prépare une solution aqueuse contenant :

- 600 ml d'eau distillée,
- 2,5 g de tensio-actif RHODOVIOL ® 25/140,
- 5,6 g de silice de précipitation TIXOSIL ® 375.

On ajoute à ce mélange séparément et dans l'ordre :

- 42 g de la composition organopolysiloxane définie à l'exemple 1, cette composition étant ajoutée à la solution aqueuse sous forte agitation pour former une émulsion,
- 5 g de NIVERGOLINE ® base en poudre, à savoir du diméthyl-1,6(bromo-5 nicotinoyloxyméthyl)-8βméthoxy-10αergoline, commercialisé par RHONE-POULENC (Laboratoire SPECIA), en solution dans 7,3 g de chloroforme.

On procède ensuite exactement suivant le mode opératoire de l'exemple 4, sauf que les billes de silicone obtenues sont en outre séchées sous vide à 60 °C pendant 10 heures. Le taux d'extractibles rapporté à la masse de composition organopolysiloxane de départ est de 7,2 %.

La granulométrie des billes est la suivante :

$\phi > 1$ mm : 23,9 %
500 µm < $\phi$ < 1 mm : 30,5 %
200 µm < $\phi$ < 500 µm : 24,6 %
100 µm < $\phi$ < 200 µm : 15,2 %
$\phi$ < 100 µm : 5,8 %

- EXEMPLE 7 :

On prépare une solution aqueuse contenant :
- 900 ml d'eau distillée,
3,6 g de tensio-actif RHODOVIOL ® 25/140 commercialisé par la Société RHONE-POULENC,
- 12 g de silice de précipitation de surface spécifique BET 160 mR2F/g.

On coule dans cette solution aqueuse, sous forte agitation pour former une émulsion :
- 100 g d'une composition d'organopolysiloxane provenant du mélange de :
- 14,5 g d'un polyorganohydrogénosiloxane porteur de fonction époxy de formule moyenne :

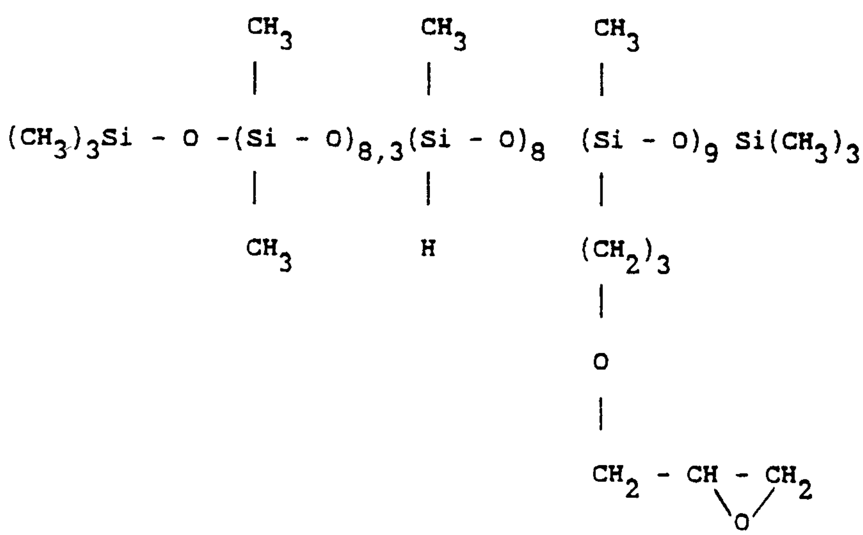

- 85,5 g d'un polydiméthylsiloxane bloqué à chaque extrémité de la chaîne par un motif diméthylvinylsiloxy et de viscosité 600 mPa.s à 25 °C,
- 15 mg d'une solution de platine dans l'hexane titrant 8,7 % de platine métal.

On porte la température de l'eau à 50 °C, on l'y maintient 15 minutes puis on la monte progressivement jusqu'à 80 °C et on laisse sous agitation pendant 1 heure 30. Ensuite on refroidit les solutions à 25 °C, on filtre les microbilles sur toiles polyester et on lave plusieurs fois à l'eau distillée pour éliminer le tensio-actif et la silice.

Ces billes sont récupérées et mélangées à quatre parties de la même silice de combustion utilisée au départ, puis placées sous vide à 125 °C pendant 3 heures. Elles sont ensuite récupérées, tamisées pour éliminer l'excès de silice et fractionnées par taille de particules.

Le rendement massique obtenu est de 90 %.

Les microbilles obtenues ont un taux d'extractibles de 2,1 % par rapport au poids de la composition.

Par passage au tamis, on détermine la répartition suivante de diamètre particulaire ($\phi$) des microcapsu-

les :
100 µm < $\phi$ < 200 µm : 19,2 %
200 µm < $\phi$ < 500 µm : 52,1 %
500 µm < $\phi$ < 1000 µm : 28,7 %

- EXEMPLE 8

On prépare une solution aqueuse contenant :
- 900 ml d'eau distillée,
- 3,62 g de tensio-actif RHODOVIOL ® 25/140 commercialisé par la Société RHONE-POULENC,
- 12 g de silice de précipitation de surface spécifique BET 160 mR2F/g.

On coule dans cette solution aqueuse, sous forte agitation pour former une émulsion :
- 100 g d'une composition d'organopolysiloxane provenant du mélange de :
- 10 g d'un polyorganohydrogénosiloxane porteur de fonction époxy de formule moyenne :

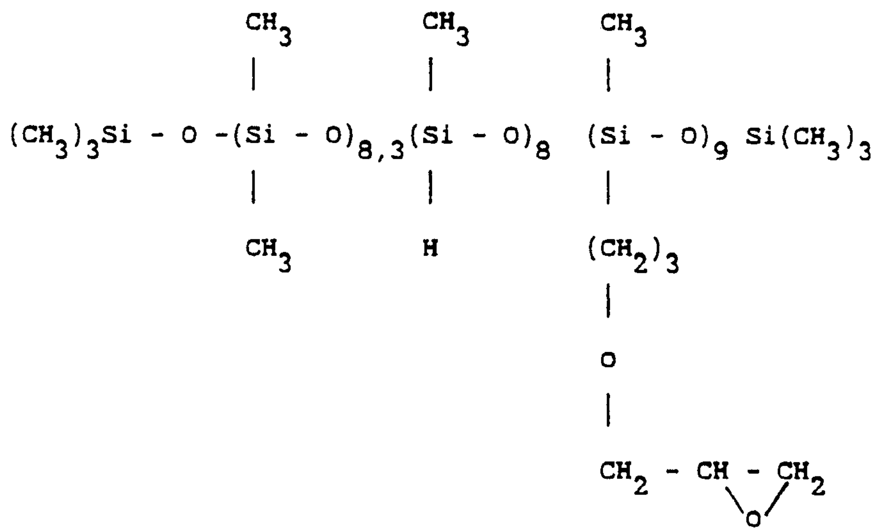

- 90 g d'un polydiméthylsiloxane porteur de fonction époxy de formule moyenne :

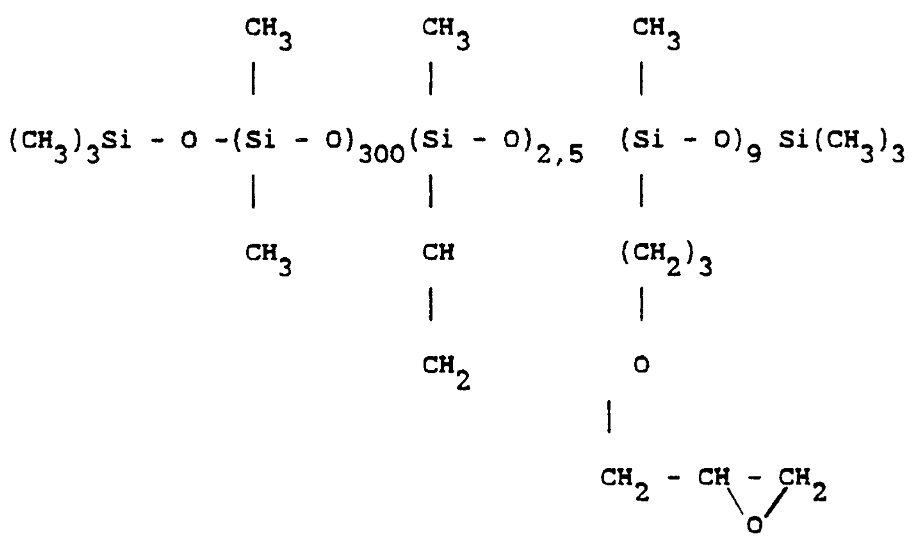

- 30 mg d'une solution de platine dans l'hexane titrant 8,7 % de platine métal.

On porte la température de l'eau à 50 °C, on l'y maintient 15 minutes puis on la monte progressivement jusqu à 80 °C et on laisse sous agitation pendant 1 heure 30 minutes. Ensuite on refroidit les solutions à 25 °C, on filtre les microbilles sur toiles polyester et on lave plusieurs fois à l'eau distillée pour éliminer le tensio-actif et la silice.

Ces billes sont récupérées et mélangées à 4 parties de la même silice de combustion utilisée au départ, puis placées sous vide à 125 °C pendant 3 heures. Elles sont ensuite récupérées, tamisées pour éliminer l'excès de silice et fractionnées par taille de particules.

Le rendement massique obtenue est de 90 %.

Les microbilles obtenues ont un taux d'extractibles de 5,2 % par rapport au poids de la composition.

Par passage au tamis, on détermine la répartition suivante de diamètre particulaire ($\phi$) des microcapsules :

2 mm > $\phi$ > 400 µm : 58 %

100 µm < $\phi$ < 400 µm : 42 %

- EXEMPLES COMPARATIFS 9, 10 ET 11 :

On répète exactement le mode opératoire des exemples 1, 2 et 3, sauf que l'on n'introduit pas de silice de précipitation.

On obtient dans les trois cas des particules collantes agglomérées entre elles et par conséquent inutilisables.

**Revendications**

1. - Procédé de préparation de particules, de granulométrie moyenne comprise entre 50 µm et 3 mm, non collantes à base d'une composition organopolysiloxane réticulée en un élastomère par des réactions de polyaddition, catactérisé en ce que :

a) - on met en émulsion :

(A) - 100 parties d'un polydiorganosiloxane portant par molécule au moins deux groupes Si-Vinyle,

(B) - un organohydrogénopolysiloxane présentant au moins trois groupe SiH par molécule en quantité telle que le rapport molaire SiH/SiVi soit compris entre 0,6 et 8, de préférence entre 0,8 et 4,

(C) - une quantité efficace d'un catalyseur au platine,

(D) - une quantité efficace d'un agent tensio-actif du type huile dans eau,

(E) - 3 à 100 parties, de préférence 5 à 30 parties d'une poudre de silice de combustion ou de précipitation, et

(F) - de l'eau,

b) - on formes les particules par chauffage à une température comprise entre 40 et 100 °C.

2. - Procédé selon la revendication 1, catactérisé en ce que l'on remplace tout ou partie du diorganopolysiloxane (A) par un diorganopolysiloxane ($A_1$) présentant en outre, par rapport au diorganopolysiloxane (A), au moins un radical organique époxy fonctionnel ayant de 4 à 20 atomes de carbone.

3 - Procédé selon l'une quelconque des revendications précédentes, caractérisé en ce que l'on remplace tout ou partie de l'organohydrogénopolysiloxane (B) par un organohydrogénopolysiloxane ($B_1$), présentant en outre, par rapport au diorganopolysiloxane (B) au moins un radical organique époxy fonctionnel ayant de 4 à 20 atomes de carbone.

4. - Procédé selon l'une quelconque des revendications précédentes, caractérisé en ce que les organopolysiloxanes (A) et (B) sont introduits dans l'eau, sous la forme d'une solution dans un solvant organique volatil dont le point d'ébullition est inférieur à 100 °C.

5 - Procédé selon l'une quelconque des revendications précédentes, caractérise en ce que l'emulsion comporte en outre un principe actif introduit sous la forme d'une solution dans le solvant organique volatil.

6. - Procédé selon l'une quelconque des revendications 1 à 3 caractérisé en ce que l'émulsion comporte en outre un principe actif (G) introduit sous la forme d'une solution dans l'eau (F) de l'émulsion.

7. - Procédé selon la revendication 6, caractérisé en ce que la solution est saturée.

8. - Procédé selon la revendication 1, caractérisé en ce que le principe actif (G) est introduit sous forme de microparticles et est dispersé au sein de la composition organopolysiloxane avant sa mise en émulsion.

9. - Utilisation des particles obtenues par la mise en oeuvre des procédés tels que définis à l'une quelconque des revendications 1 à 8, comme charge pour matrice polymère naturelle ou synthétique.

Office européen des brevets

# RAPPORT DE RECHERCHE EUROPEENNE

Numero de la demande

EP 90 42 0025

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 242 219 (TORAY SILICONE CO.) * Revendications 1-5; pages 9,10; exemple 1 * --- | 1-9 | C 08 J 3/03<br>C 08 J 3/12 //<br>A 61 K 9/16<br>C 08 L 83:06 |
| A | EP-A-0 277 740 (DOW CORNING CORP.) * Revendications 1-7; page 6, exemple 2 * --- | 1,8,9 | |
| A | EP-A-0 304 946 (TORAY SILICONE CO.) * Revendications 1-3; page 10, exemple 6 * ----- | 1,9 | |
| | | | **DOMAINES TECHNIQUES RECHERCHES (Int. Cl.5)** |
| | | | C 08 J<br>A 61 K |

Le présent rapport a été établi pour toutes les revendications

| Lieu de la recherche | Date d'achèvement de la recherche | Examinateur |
|---|---|---|
| LA HAYE | 11-04-1990 | OUDOT R. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)